# EUROPEAN PATENT APPLICATION

(11) **EP 4 667 013 A1**
(43) Date of publication of application: **24.12.2025**
(21) Application number: 24756054.3
(22) Date of filing: 04.02.2024
(51) Int. Cl.: A61K 38/17, A61P 19/10, C07K 14/47, C07K 1/12

(54) **DRUG FOR TREATING OSTEOPOROSIS**

(30) Priority: 15.02.2023 CN 202310118563
(71) Applicant: Shanghai Seme Cell Technology Co., Ltd, Shanghai 200241 (CN)
(72) Inventor: ZHANG, Wenjie, Shanghai 200241 (CN); WANG, Yahui, Shanghai 200241 (CN)
(74) Representative: Mewburn Ellis LLP
(86) International application number: PCT/CN2024/075736
(87) International publication number: WO 2024/169680

(57) **Abstract**

A drug for treating osteoporosis. Provided is the use of annexin A5 in the preparation of a composition or preparation having one or more uses selected from the group consisting of: (i) preventing and/or treating osteoporosis; (ii) inhibiting osteoclast activity; and (iii) reducing bone loss. The annexin A5 can prevent and/or treat osteoporosis by means of inhibiting osteoclast activity and preventing bone loss.

## Description

### FIELD OF THE INVENTION

The present invention relates to the field of medicine, particularly to a drug for treating osteoporosis.

### BACKGROUND OF THE INVENTION

Osteoporosis is a systemic bone disease characterized by a decrease in bone density and quality due to various reasons, and destruction of bone microstructure, leading to an increased bone fragility and a heightened susceptibility to fractures. Osteoporosis can directly or indirectly cause pain, spinal deformation, and fractures. Pain symptoms can significantly reduce the life quality of patients. Spinal deformation and fractures can cause disability, making patients unable to move independently, increasing the incidence of other complications, and resulting in great physical and mental burden.

At present, anti-bone resorption drugs, bone formation promoters, calcium supplements, and other vitamins are mainly used in clinical practice. However, therapeutic drugs have significant side effects and limited application, and the effect of adjuvant drug therapy is not significant.

Therefore, there is a need in this field to develop a drug for treating osteoporosis.

### SUMMARY OF THE INVENTION

The object of the present invention is to provide a use of annexin A5 for preventing and/or treating osteoporosis.

In the first aspect of the present invention, provided is a use of annexin A5 in the preparation of a composition or formulation, wherein the composition or formulation is used for one or more uses selected from the group consisting of: (i) preventing and/or treating osteoporosis; (ii) inhibiting osteoclast activity; and (iii) reducing bone loss.

In another preferred embodiment, the osteoporosis is selected from the group consisting of: primary osteoporosis, secondary osteoporosis, and the combination thereof.

In another preferred embodiment, the primary osteoporosis includes one or more of postmenopausal osteoporosis (type I), geriatric osteoporosis (type II), and idiopathic osteoporosis (adolescent type).

In another preferred embodiment, the secondary osteoporosis includes one or more of osteoporosis caused by endocrine diseases, osteoporosis caused by connective tissue diseases, osteoporosis caused by kidney diseases, osteoporosis caused by gastrointestinal diseases, osteoporosis caused by drugs, and osteoporosis caused by malnutrition.

In another preferred embodiment, the osteoporosis includes osteoporosis caused by estrogen deficiency.

In another preferred embodiment, the osteoporosis caused by estrogen deficiency includes postmenopausal osteoporosis and osteoporosis caused by ovarian injury.

In another preferred embodiment, the preventing and/or treating osteoporosis comprises one or more features selected from the group consisting of:
(i) inhibiting osteoclast activity;
(ii) reducing bone loss;
(iii) increasing bone volume fraction;
(iv) increasing the number of bone trabeculae;
(v) increasing bone connectivity density; and
(vi) reducing the degree of bone trabeculae separation.

In another preferred embodiment, the preventing and/or treating osteoporosis does not include one or more of promoting the release of matrix vesicles during bone mineralization, promoting osteoblast expression, or promoting the expression of osteogenic differentiation markers.

In another preferred embodiment, the preventing and/or treating osteoporosis refers to protecting bone from loss by inhibiting osteoclast activity, thereby preventing and/or treating osteoporosis.

In another preferred embodiment, the bone is selected from the group consisting of: tibia, femur, cervical vertebrae, lumbar vertebrae, thoracic vertebrae, knee joint bones, hip joint bones, and the combinations thereof.

In another preferred embodiment, the composition or formulation contains 0.1-99.9wt% of annexin A5, preferably 1-99.9wt%, preferably 20-99.9wt% of annexin A5, such as 30wt%, 40wt%, 50wt%, 60wt%, 70wt%, 80wt%, 90wt%, 95wt%, based on the total weight of the composition or formulation.

In another preferred embodiment, the annexin A5 is extracted from adipose tissue.

In another preferred embodiment, the adipose tissue comprises heterogeneous adipose tissue, allogeneic adipose tissue, or homologous adipose tissue.

In another preferred embodiment, the composition or formulation comprises a pharmaceutical composition or formulation, a food composition or formulation, a health care composition or formulation, or a dietary supplement.

In another preferred embodiment, the composition or formulation further comprises acceptable carriers in pharmaceuticals, food, health care products, or dietary products.

In another preferred embodiment, the dosage form of the composition or formulation is an oral formulation, an external formulation, or an injection formulation.

In another preferred embodiment, the injection formulation is an intravenous injection, an intraperitoneal injection, or an intramuscular injection.

In another preferred embodiment, the dosage form of the composition or formulation is a solid dosage form, a semi-solid dosage form, or a liquid dosage form.

In another preferred embodiment, the dosage form of the composition or formulation is powders, granules, capsules, injections, tinctures, oral liquids, tablets or lozenges.

In another preferred embodiment, the composition or formulation is administered orally, topically, intraperitoneally, intravenously, or subcutaneously.

In the second aspect of the present invention, provided is a method for (i) preventing and/or treating osteoporosis; (ii) inhibiting osteoclast activity; and/or (iii) reducing bone loss comprising the step of administering annexin A5 to a subject in need thereof.

In another preferred embodiment, the subject is a human or a non-human mammal.

In another preferred embodiment, the non-human mammal includes a pig, a cow, a sheep, a rat, a mouse, or a rabbit.

In another preferred embodiment, the subjects are of various age groups.

In another preferred embodiment, the subject is a subject who have already developed, are currently developing, or are about to develop osteoporosis.

In the third aspect of the present invention, provided is an *in vitro* non-therapeutic and non-diagnostic method for inhibiting osteoclast activity, comprising the step of administering annexin A5 to osteoclasts.

It should be understood that within the scope of the present invention, the above-described technical features of the present invention and the technical features described in detail below (e.g., examples) may be combined with each other to constitute a new or preferred technical solution. Limited by space, it will not be repeated here.

### DETAILED DESCRIPTION OF THE INVENTION

After extensive and in-depth research, the inventor unexpectedly discovered that annexin A5 can effectively protect bone from loss by inhibiting osteoclast activity, thereby preventing and/or treating osteoporosis. The present invention has been completed on this basis.

### Terms

Unless otherwise defined, all technical and scientific terms used herein have the same meanings as commonly understood by those of ordinary skill in the art to which the present invention belongs.

As used herein, the terms "comprise", "include", and "contain" are used interchangeably to include not only closed definitions, but also semi-closed, and open definitions. In other words, the terms include "consist of" and "substantially consist of".

As used herein, when referring to specific listed values, the term "about" means that the value can vary by no more than 1% from the listed values. For example, as used herein, the expression "about 100" includes all values between 99 and 101 (e.g. 99.1, 99.2, 99.3, 99.4, etc.).

The term "prevent" and "treat" described in the present invention include delaying and terminating the progression of diseases, and do not require 100% inhibition, elimination, and reversal. In some embodiments, the composition or formulation of the present invention prevents, reduces, inhibits, and/or reverses osteoporosis by at least about 10%, at least about 30%, at least about 50%, or at least about 80% compared to levels observed in the absence of the composition or formulation of the present invention.

### Annexin

Annexins are a widely distributed class of calcium-dependent phospholipid-binding proteins. Based on molecular phylogenetics and comparative genomics analysis, this protein family is uniformly named annexin (abbreviated as Anx), followed by capital letters to denote different species: A, B, C, D, E, which represent vertebrates, invertebrates, fungi and slime molds, plants, and protists, respectively. Up to now, the Anx A family has a total of 12 members: A1-A11 and A13 (A12 not specified).

Representatively, the annexin include annexin A5. Annexin A5 (AnxA5) is widely distributed in various tissues and cells of the body. *In vitro* studies have found that AnxA5 has multiple functions such as anti-phospholipase, anticoagulation, and anti-kinase, but its specific *in vivo* functions are not very clear. Currently, it is mainly used as a reagent for detecting cell apoptosis.

The annexin that can be used in the present invention is not particularly limited and can be annexin derived from any organism, preferably from mammals (such as primates), and more preferably from humans. In addition, it should be understood that "annexin" include a wild-type or a mutant (including truncated) annexin, as long as the mutant annexin retains or maintains the detoxification activity of the wild-type annexin.

### Use

The present invention provides a use of annexin A5 in the preparation of a composition or formulation, wherein the composition or formulation is used for one or more uses selected from the group consisting of: (i) preventing and/or treating osteoporosis; (ii) inhibiting osteoclast activity; and (iii) reducing bone loss.

In the present invention, the osteoporosis includes osteoporosis caused by estrogen deficiency, such as postmenopausal osteoporosis and osteoporosis caused by ovarian injury.

In another preferred embodiment, the preventing and/or treating osteoporosis comprises one or more features selected from the group consisting of:
(i) inhibiting osteoclast activity;
(ii) reducing bone loss;
(iii) increasing bone volume fraction;
(iv) increasing the number of bone trabeculae;
(v) increasing bone connectivity density; and
(vi) reducing the degree of bone trabeculae separation.

In the present invention, annexin A5 achieves the effect of preventing and/or treating osteoporosis by inhibiting osteoclast activity and preventing bone loss.

In prior art, it has been mentioned that promoting bone formation through mediating the release of matrix vesicles during bone mineralization belongs to the category of promoting bone formation. That is different from reducing bone loss by inhibiting osteoclast activity in the present application.

### Compositions and administration

The compositions as described in the present invention include, but are not limited to, pharmaceutical compositions, food compositions, health care compositions, dietary supplements, and the like.

Representatively, the annexin of the present invention can be prepared as pharmaceutical compositions in dosage forms such as tablets, capsules, powders, microgranules, solutions, lozenges, jellies, creams, spirits, suspensions, tinctures, poultice, liniment, lotions, and aerosols, etc. Pharmaceutical compositions can be prepared by commonly known preparation techniques, and suitable pharmaceutical additives can be added to the drug.

The compositions of the present invention can also include acceptable carriers in pharmaceuticals, food, health care products, or dietary products. "Acceptable carriers in pharmaceuticals, food, health care products, or dietary products" mean one or more compatible solid or liquid filler or gel substances that are suitable for human use and must have sufficient purity and sufficiently low toxicity. "Compatibility" herein refers to the ability of the components of a composition to blend with the compounds of the invention and with each other without significantly reducing the efficacy of the compounds. Examples of acceptable carriers in pharmaceuticals, food, health care products, or dietary products include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as Tween^{®}), wetting agents (such as sodium dodecyl sulfate), colorants, flavoring agents, stabilizers, antioxidants, preservatives, pyrogen-free water, etc.

The administration modes of the compositions of the present invention are not particularly limited, and representative modes of administration include (but are not limited to) oral, parenteral (intravenous, intramuscular), topical administration, preferably injection administration.

Representatively, solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following components:(a) fillers or compatibilizers, e.g., starch, lactose, sucrose, glucose, mannitol and silicic acid; (b) binders, e.g., hydroxymethylcellulose, alginate, gelatin, polyvinylpyrrolidone, sucrose, and gum arabic; (c) humectants, e.g., glycerol; (d) disintegrants, e.g., agar, calcium carbonate, potato starch or tapioca starch, alginic acid, certain composite silicates, and sodium carbonate; (e) dissolution-retarding agents, e.g., paraffin; (f) absorption accelerators, e.g., quaternary amine compounds; (g) wetting agents, e.g., cetyl alcohol and glycerol monostearate; (h) adsorbents, e.g., kaolin; and (i) lubricants, e.g., talc, calcium stearate, magnesium stearate, solid polyethylene glycol, sodium dodecyl sulfate, and the mixtures thereof. In capsules, tablets and pills, dosage forms may also contain buffers.

Liquid dosage forms for oral administration include pharmaceutically acceptable emulsions, solutions, suspensions, syrups or tinctures. In addition to the active compounds, the liquid dosage forms may contain inert diluents conventionally used in the art such as water or other solvents, solubilizers and emulsifiers such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or the mixtures thereof and the like.

In addition to these inert diluents, the compositions may also contain auxiliaries such as wetting agents, emulsifiers, suspending agents, sweeteners, flavoring agents and spices.

In addition to the active ingredient, the suspensions may comprise suspending agents, such as ethoxylated isooctadecanol, polyoxyethylene sorbitol and dehydrated sorbitol esters, microcrystalline cellulose, methanolic aluminum, agar, and the mixtures thereof.

The compositions for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compounds of the present invention for external administration include ointments, powders, patches, sprays and inhalants. The active ingredient is mixed with physiologically acceptable carriers and any preservatives, buffers or propellants that may be required if necessary under sterile conditions.

The annexin of the present invention can be administered alone, or in combination with other drugs for preventing and/or treating osteoporosis and/or complications thereof, such as vitamin D, bisphosphonates, raloxifene, Denosumab, Teriparatide, Abaloparatide, and Romosozumab.

When the composition is administered, a safe and effective amount of the cell-free fat extract of the present invention is administrated to a human or a non-human animal (e.g., a rat, a mouse, a dog, a cat, a cow, a chicken, a duck, etc.) in need of treatmentat, wherein the dose when administered is an effective administration dosage which is acceptable in pharmaceuticals, food, health care products, or dietary products. As used herein, the term "safe and effective amount" refers to an amount that produces function or activity to humans and/or animals and is acceptable to humans and/or animals. Those ordinary of skill in the art will understand that the "safe and effective amount" may vary depending on the form of the pharmaceutical composition, the route of administration, the excipient of the drug used, the severity of the disease, and the combination with other drugs. For example, for a person with a body weight of 60kg, the daily dose is usually 0.1 to 1000 mg, preferably 1 to 600 mg, more preferably 2 to 300 mg. Of course, the specific dosage should also consider the route of administration, the patient's health status and other factors, which are all within the scope of skilled physician skills.

### The main advantages of the present invention include:

The inventors have discovered for the first time that annexin A5 can prevent and/or treat osteoporosis by inhibiting osteoclast activity and preventing bone loss.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

Annexin A5: extracted and purified from human adipose tissue, accession number: P08758.

### Example 1

### 1. Animal experiment

### Model establishment

24 of 8-week-old female SD rats were randomly divided into three groups, with 8 rats in each group: blank control group (Sham group), PBS control group (OVX+PBS group), and experimental group (OVX+A5 group). The OVX groups underwent bilateral oophorectomy, while the Sham group underwent sham surgery.

### Experimental Method

After 8 weeks of surgery, 8 rats in the OVX+PBS group were intraperitoneally injected with PBS (1.5mg/kg) once every two days for 3 consecutive weeks, for a total of 12 injections; 8 rats in the OVX+A5 group were intraperitoneally injected with annexin A5 (1.5mg/kg) once every two days for 3 consecutive weeks, for a total of 12 injections; Sham group does not receive special treatment. After 12 treatments, all rats were euthanized and their bone samples were collected for subsequent evaluation.

High-resolution micro CT analysis were performed on the collected right femur and tibia, and the evaluation parameters of bone microstructure were quantified using Scanco software.

### 2. Cell experiment

### Model establishment

Bone marrow was extracted from 4-week-old female SD rats, and bone marrow mononuclear macrophages (BMMs) were isolated, and cultured in α-MEM medium containing 25ng/mL M-CSF, 10% fetal bovine serum, and 1% tertiary antibody. The culture medium was changed every 3 days until BMMs reached 95% fusion under the microscope before passaging. The digested BMMs were placed in intact α-MEM medium containing 50ng/mL RANKL and 25ng/mL M-CSF, and inoculated into a 24-well plate at a cell concentration of 3*10⁴/mL. Induction culture lasted for 7 days, with the medium replaced every 3 days.

### Experimental Method

After successful induction of osteoclasts, the collected cells were placed in a 96-well plate and divided into a blank control group (intact α-MEM medium) and an experimental group (intact α-MEM medium with different concentrations of annexin A5 added). After 7 days of cultivation, the cells were fixed for tartrate resistant acid phosphatase (TRAP) staining. TRAP-stained images were obtained using an optical microscope and TRAP+osteoclast regions in each sample was quantified using Image Pro Plus software.

### 3. Statistical analysis

GraphPad. Prism. v5.0 was used for data analysis, "*" p<0.05, "**" p<0.01, "***" p<0.001.

### 4. Experimental results

In the rat OVX model, annexin A5 successfully protected the rat's bone from loss; The cell experiment results showed that annexin A5 successfully inhibited osteoclast activity.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A use of annexin A5 in the preparation of a composition or formulation, wherein the composition or formulation is used for one or more uses selected from the group consisting of: (i) preventing and/or treating osteoporosis; (ii) inhibiting osteoclast activity; and (iii) reducing bone loss.

2. The use of claim 1, wherein the osteoporosis is selected from the group consisting of: primary osteoporosis, secondary osteoporosis, and the combination thereof.

3. The use of claim 1, wherein the osteoporosis includes osteoporosis caused by estrogen deficiency.

4. The use of claim 3, wherein the osteoporosis caused by estrogen deficiency includes postmenopausal osteoporosis and osteoporosis caused by ovarian injury.

5. The use of claim 1, wherein the annexin A5 is extracted from adipose tissue.

6. The use of claim 1, wherein the adipose tissue comprises heterogeneous adipose tissue, allogeneic adipose tissue, or homologous adipose tissue.

7. The use of claim 1, wherein the preventing and/or treating osteoporosis refers to protecting bone from loss by inhibiting osteoclast activity, thereby preventing and/or treating osteoporosis.

8. The use of claim 1, wherein the dosage form of the composition or formulation is an oral formulation, an external formulation, or an injection formulation.

9. The use of claim 1, wherein the composition or formulation is administered orally, topically, intraperitoneally, intravenously, or subcutaneously.

10. An *in vitro* non-therapeutic and non-diagnostic method for inhibiting osteoclast activity, comprising the step of administering annexin A5 to osteoclasts.
